# EUROPEAN PATENT SPECIFICATION

(11) **EP 2 717 840 B1**
(45) Date of publication and mention of the grant of the patent: **08.03.2017**
(21) Application number: 12721562.2
(22) Date of filing: 22.05.2012
(51) Int. Cl.: A61K 8/42, A61K 8/44, A61K 8/46, A61Q 5/02, A61Q 5/12, A61Q 5/00

(54) **HAIR CLEANSING COMPOSITION**
HAARREINIGUNGSMITTELZUSAMMENSETZUNG
COMPOSITION DE NETTOYAGE POUR LE CHEVEUX

(30) Priority: 10.06.2011 EP 11169430
(43) Date of publication of application: 16.04.2014
(73) Proprietor: Kao Germany GmbH, 64297 Darmstadt (DE)
(72) Inventor: MOLENDA, Michael, 60318 Frankfurt (DE); LIPINSKI, Normen, 60325 Frankfurt (DE)
(86) International application number: PCT/EP2012/059430
(87) International publication number: WO 2012/168060

(56) References cited:
- EP-A1- 1 925 290
- EP-A2- 2 033 624
- WO-A1-98/32416
- WO-A1-2005/120442
- DE-C1- 19 737 604
- US-A- 5 415 810
- US-A- 6 013 616
- US-A1- 2003 172 474
- LOEFFLER M: "OPTIMIERTES TENSIDKONZENTRAT ENTHALTEND BETAIN, TAURAT UND ISETHIONAT", SOFW-JOURNAL SEIFEN, OELE, FETTE, WACHSE, VERLAG FUR CHEMISCHE INDUSTRIE, AUGSBURG, DE, vol. 133, no. 9, 6 September 2007 (2007-09-06), XP001542782, ISSN: 0942-7694

## Description

Present invention relates to an aqueous cleansing composition for keratin fibres, especially human hair, comprising at least one mono ester anionic surfactant, at least one anionic amide surfactant and at least one amphoteric amide surfactant.

Cleansing compositions have been known for many years. Many patent applications and scientific publications deal with such compositions aiming at cleansing, especially improved conditioning effects after cleansing. Cleansing compositions wash out dirt on the hair and at the same time dyestuffs deposited onto or into hair are also washed out, which leads to shorter colour durability after coloration services and is not economical in terms of frequent hair dressing need and also health of hair. Attempts have been made to reduce colour wash out from coloured hair with various ways. One of them is so called colour sealing which requires application of an additional mostly hydrophobic composition right after colouring hair so that the dyes are not easily solubilised by the high surfactant content of cleansing compositions. The other approach has been to use a special surfactant combination which interacts with dyestuff molecules and also with hair in a lesser extent. None of the approaches are optimal for variably coloured hair and, therefore, there is a great need for further improvements in the field.

Aim of the present invention is to provide an aqueous cleansing composition having optimal benefits of foam properties in terms of its volume and creaminess as well as improved conditioning effects on keratin fibres, especially human hair, in terms of combability, smoothness, elasticity, softness, volume and body and at the same time washes out artificial hair colour in a lesser extend so that the coloured hair keeps its colour and therefore shine and healthy appearance.

Present inventors have surprisingly found that an aqueous cleansing composition comprising at least one mono ester anionic surfactant, at least one anionic amide surfactant and at least one amphoteric amide surfactant provides excellent foam and conditioning properties and also washes out less colour from hair so that long lasting colours are achieved.

Accordingly, the first object of the present invention is an aqueous cleansing composition comprising at least one mono ester anionic surfactant selected from the compounds according to the general structure

R₃₀ C(O) O R₃₁ SO₃ M

wherein R₃₀ is a saturated or unsaturated, straight or branched alkyl chain with 9 to 17 C atoms, R₃₁ is a methyl or straight or branched alkyl chain with 2 to 4 C atoms and M is ammonium or an alkali metal, preferably sodium, at least one anionic amide surfactant selected from the compounds according general structure 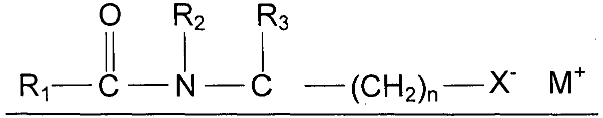 wherein R₁ is a saturated or unsaturated, straight or branched alkyl chain with 7 to 17 C atoms, R₂ is H, R₃ is H, COO⁻ M⁺, CH₂COO⁻ M or COOH, n is 0 to 2, X is COO⁻ or SO₃⁻ and M is independent from each other H, sodium, potassium or ammonium and at least one amphoteric amide surfactant, wherein the composition has a pH in the range of 2.5 to 6.5.

With the term mono ester anionic surfactant it is meant that the anionic surfactant includes only one ester group in its molecule.

With the term anionic amide surfactant it is meant that the anionic surfactant comprise at least one amide group in its molecule and especially those surfactants are meant derived from taurate, glutamate and aspartate.

With the term amphoteric amide surfactant it is meant that the surfactant is amphoteric in the composition it is comprised and it includes at least one amide group in its molecule.

Second object of the present invention is the use of cleansing composition of the present invention for cleansing and conditioning hair.

Third objective of the present invention is the use of cleansing composition of the present invention for reducing colour fading and/or wash out from artificially coloured hair.

Further object of the present invention is a kit for treating hair wherein it comprises two or more compositions wherein at least one of the compositions is a composition according to present invention.

Still further object of the present invention is a process for treating hair wherein hair is artificially coloured in a first step and washed with a cleansing composition of the present invention.

Compositions of the present invention comprises surfactant at a total concentration of 5 to 50%, preferably 7.5 to 40%, more preferably 8 to 30% and most preferably 10 to 25% and particularly 10 to 20% by weight, calculated to the total of the composition.

Suitable examples to mono ester anionic surfactant are sodium cocoyl isethionate, sodium lauroyl isethionate, sodium oleoyl isethionate, sodium oleoyl methyl isethionate, sodium lauroyl methyl isethionate, sodium stearoyl isethionate, sodium stearoyl methyl isethionate and ammonium cocoyl isethionate. Preferred are sodium cocoyl isethionate and ammonium cocoyl isethionate.

Concentration of at least one mono ester anionic surfactant is in the range of 1 to 15%, preferably 2 to 12%, more preferably 3 to 10% and most preferably 3 to 7.5% by weight calculated to the total content of the composition. The concentrations mentioned here are total concentration ranges in case more than one mono ester anionic surfactant is present.

Composition of the present invention comprises at least one anionic amino acid surfactant of the above general structure. In the preferred embodiment of the present invention R₁ is a saturated or unsaturated, straight or branched alkyl chain with 9 to 17 C atoms, and more preferably 9 to 13 C atoms, R₂ is H, R₃ is H, COO⁻ M⁺, CH₂COO⁻ M or COOH, n is 0 to 2, X is COO⁻ or SO₃⁻ and M is independent from each other H, sodium, potassium or ammonium. It should be noted that alkyl chain includes also mixture of various alkyl groups as present especially in plant triglyceride derived alkyl chains such as cocoyl chain.

Cleansing composition of the present invention comprises at least one anionic amino acid surfactant according to the general formula of above at a concentration of 1 to 15%, preferably 2 to 12%, more preferably 3 to 10% and most preferably 3 to 7.5% by weight calculated to the total content of the composition. The concentrations mentioned here are total concentration ranges in case more than one amino acid surfactant is present.

Suitably amino acid surfactant types are taurate, glutamate, aspartate surfactants, and mixtures thereof. Preferred are taurate and glutamate surfactants and mixtures thereof. Most preferred is glutamate type surfactants.

Suitable taurate surfactants are according to the general formula 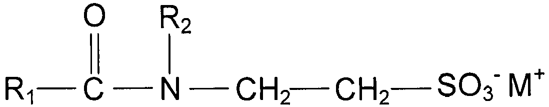 wherein R₁ is preferably a saturated or unsaturated, straight or branched alkyl chain with 7 to 17 C atoms, and more preferably 9 to 13 C atoms, R₂ is H or methyl, and M is H, sodium or potassium. Suitable examples are potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, and sodium methyl stearoyl taurate and mixtures thereof. Preferred are potassium cocoyl taurate, potassium methyl cocoyl taurate, sodium caproyl methyl taurate, sodium cocoyl taurate, sodium lauroyl taurate, sodium methyl cocoyl taurate and sodium methyl lauroyl taurate and mixtures thereof. More preferred are potassium cocoyl taurate, potassium methyl cocoyl taurate, sodium cocoyl taurate, sodium lauroyl taurate, sodium methyl cocoyl taurate and sodium methyl lauroyl taurate and mixtures thereof.

Suitable glutamate surfactants are according to the general formula 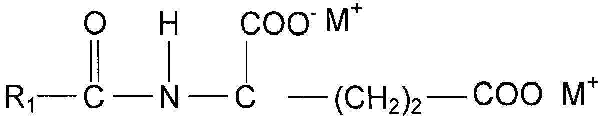 wherein R₁ is preferably a saturated or unsaturated, straight or branched alkyl chain with 7 to 17 C atoms, and more preferably 9 to 13 C atoms, and M is independent from each other H, sodium or potassium. Suitable examples are dipotassium capryloyl glutamate, dipotassium undecylenoyl glutamate, disodium capryloyl glutamate, disodium cocoyl glutamate, disodium lauroyl glutamate, disodium stearoyl glutamate, disodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, and sodium undecylenoyl glutamate and mixtures thereof. Preferred are disodium capryloyl glutamate, disodium cocoyl glutamate, disodium lauroyl glutamate, potassium capryloyl glutamate, potassium cocoyl glutamate, potassium lauroyl glutamate, potassium myristoyl glutamate, sodium capryloyl glutamate, sodium cocoyl glutamate, sodium lauroyl glutamate, and sodium myristoyl glutamate and mixtures thereof. More preferred are disodium capryloyl glutamate, disodium cocoyl glutamate, disodium lauroyl glutamate, potassium capryloyl glutamate, potassium cocoyl glutamate, potassium lauroyl glutamate, sodium capryloyl glutamate, sodium cocoyl glutamate, and sodium lauroyl glutamate and mixtures thereof.

Suitable glycine surfactants are according to the general formula 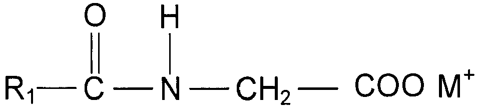 wherein R₁ is preferably a saturated or unsaturated, straight or branched alkyl chain with 7 to 17 C atoms, and more preferably 9 to 13 C atoms, and M is H, sodium or potassium. Suitable examples are palmitoyl glycine, sodium lauroyl glycine, sodium cocoyl glycine, sodium myristoyl glycine, potassium lauroyl glycine, and potassium cocoyl glycine and mixtures thereof. sarcosinate, and sodium palmitoyl sarcosinate and mixtures thereof. Preferred are potassium lauroyl sarcosinate, potassium cocoyl sarcosinate, sodium cocoyl sarcosinate, and sodium lauroyl sarcosinate and mixtures thereof. More preferred are sodium cocoyl sarcosinate, and sodium lauroyl sarcosinate and mixtures thereof.

Suitable aspartate surfactants are according to the general formula 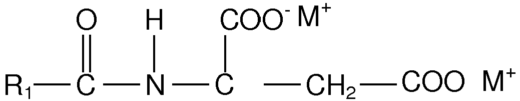 wherein R₁ is preferably a saturated or unsaturated, straight or branched alkyl chain with 7 to 17 C atoms, and more preferably 9 to 13 C atoms, and M is independent from each other H, sodium or potassium. Suitable examples are sodium lauroyl aspartate, sodium myristoyl aspartate, sodium cocoyl aspartate, sodium caproyl aspartate, disodium lauroyl aspartate, disodium myristoyl aspartate, disodium cocoyl aspartate, disodium caproyl aspartate, potassium lauroyl aspartate, potassium myristoyl aspartate, potassium cocoyl aspartate, potassium caproyl aspartate, dipotassium lauroyl aspartate, dipotassium myristoyl aspartate, dipotassium cocoyl aspartate, and dipotassium caproyl aspartate and mixtures thereof. Preferred are sodium lauroyl aspartate, sodium myristoyl aspartate, sodium cocoyl aspartate, and sodium caproyl aspartate and mixtures thereof.

It should be noted that compositions of the present invention can also comprise mixture of several type of amino acid surfactants such as mixture of glutamate and taurate surfactants, or mixture of taurate, glutamate and sarcosinate surfactants etc.

Composition of the present invention comprises at least one amphoteric amide surfactant. Suitable and preferred one are alkyl amido alkyl betaines according to the general structure 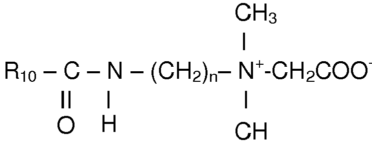 wherein R₁₀ is a straight or branched, saturated or unsaturated C₈-C₂₂-alkyl group, preferably C₁₀-C₁₈-alkyl group and more preferably C₁₀-C₁₄-alkyl group and n is 1 to 3.

Suitable amide amphoteric surfactants are alkylamidoalkyletaines such as lauramidopropyl betaine, decylamidopropyl betaine, myristylamidopropyl betaine behenamidopropyl betaine, lauramidoethyl betaine, oleylamidopropyl betaine, palmitamidopropyl betaine and cocamidopropylbetaine which is the most preferred amide amphoteric surfactant.

Concentration of at least one amide amphoteric surfactant is in the range of 1 to 15%, preferably 2 to 12%, more preferably 3 to 10% and most preferably 3 to 7.5% by weight calculated to the total content of the composition. The concentrations mentioned here are total concentration ranges in case more than one amide amphoteric surfactant is present.

Composition of the present invention comprises additionally at least one non-ionic surfactant. Nonionic surfactants are comprised at a concentration of 1 % to 15 %, preferably from 1 % to 10 % by weight, calculated to the total composition.

Nonionic surfactants preferred in the cleansing compositions according to the invention are alkyl polyglucosides of the general formula

R₈-O-(R₉O)ₙ-Zₓ,

wherein R₈ is an alkyl group with 8 to 18 carbon atoms, R₉ is an ethylene or propylene group, Z is a saccharide group with 5 to 6 carbon atoms, n is a number from 0 to 10 and x is a number between 1 and 5. Especially suited examples are decyl polyglucoside, cocoyl polyglucoside and lauryl polyglucoside.

Further nonionic surfactants are long-chain fatty acid dialkanolamides, such as coco fatty acid diethanolamide and myristic fatty acid diethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx^{®}", "Aromox^{®}" or "Genaminox^{®}".

Further nonionic surfactants useful in the compositions according to invention are C₁₀-C₂₂-fatty alcohol ethoxylate. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":
The average degree of ethoxylation thereby ranges between about 2.5 and about 25, preferably about 10 and about 20.

The more preferred non-ionic surfactants are alkyl polyglucosides such as decyl, cocoyl polyglucoside and ethoxylated fatty alcohols such as laureth-16. Especially preferred are alkyl polyglucosides such as decyl, cocoyl polyglucoside.

Composition of the present invention may comprise additional anionic and amphoteric surfactants. In principal any anionic surfactant other than mentioned above is suitable within the meaning of the present invention. Nonlimiting examples are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono-and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₇-(C₂H₄O)ₙ-O-CH₂COOX,

wherein R₇ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 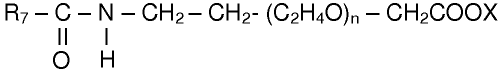 wherein R and X have the above meanings, and n is in particular a number from 1 to 10, preferably 2.5 to 5.

Such products have been known for some time and are on the market, for example, under the trade name "AKYPO^{®}" and "AKYPO-SOFT^{®}".

The most preferred as an additional anionic surfactants within the meaning of the present invention are those of alkyl ether sulphates such as lauryl ether sulphate sodium salt.

Suitable additional amphoteric surfactants are as such are in particular the various known betaines such as alkyl betaines, and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In a further preferred embodiment, cleansing composition of the present invention comprises hair-conditioning agents. Conditioning agents can be selected from oily substances, non-ionic substances, cationic amphiphilic ingredients, cationic polymers other than the one described above or their mixtures.

Oily substances are selected from such as silicone oils, either volatile or non-volatile, natural and synthetic oils. Among silicone oils those can be added to the compositions include dimethicone, dimethiconol, polydimethylsiloxane, DC fluid ranges from Dow Corning, arylated silicones such as phenyl trimethicone or any other silicone with up to 5 aryl, preferably phenyl, group in its molecule such as trimethyl pentaphenyl trisiloxane, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents can be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁₁ CO (O CH₂ CH₂)ₙ OH

or

R₁₁ CO (O CH₂ CH₂)ₙ O OC R₁₂

where R₁₁ and R₁₂ are independent from each other saturated, unsaturated or branched or non-branched alkyl chain with 7 to 21 C atoms and n is typically 2 - 100.

In one of the preferred form of the present invention, cleansing compositions comprise at least one additional cationic polymer as a conditioning agent. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, and Polyquaternium 86.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum an its derivatives known with INCl name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 67 and polyquaternium 70.

The cationic polymers also include the quaternized products of graft polymers from organopolysiloxanes and polyethyl oxazolines described in EP-A 524 612 and EP-A 640 643.

Composition of the present invention comprises cationic polymer at a concentration of 0.01 to 5%, preferably 0.02 to 4%, more preferably 0.05 to 3% and most preferably 0.1 to 2.5% by weight calculated to total composition.

Although less preferred, cleansing compositions of the present invention may comprise additionally one or more cationic surfactant(s) as conditioner according to the general formula 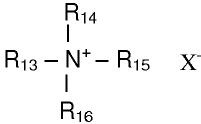 where R₁₃ is a saturated or unsaturated, branched or non-branched alkyl chain with 8-24 C atoms or

R₁₇ CO NH (CH₂)n

where R₁₇ is saturated or unsaturated, branched or non-branched alkyl chain with 7-21 C atoms and n has value of 1 - 4,
or

R₁₈ CO O (CH₂)ₙ

where R₁₈ is saturated or unsaturated, branched or non-branched alkyl chain with 7-21 C atoms and n has value of 1 - 4, and
R₁₄ is unsaturated or saturated, branched or non-branched alkyl chain with 1 - 24 C atoms or

R₁₇ CO NH (CH₂)ₙ

or

R₁₈ CO O (CH₂)ₙ

where R₁₇, R₁₈ and n are same as above.

R₁₅ and R₁₆ are lower alkyl chain with 1 to 4 carbon atoms which may be substituted with one or more hydroxyl groups, and X is anion such as chloride, bromide, methosulfate.

Typical examples of those ingredients are cetyl trimethly ammonium chloride, stear trimonium chloride, behentrimonium chloride, stearamidopropyl trimonuim chloride, dioleoylethyl dimethyl ammonium methosulfate, dioleoylethyl hydroxyethylmonium methosulfate.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, silicone oil and derivatives and cationic surfactants is in the range of 0.01 to 5% by weight, preferably 0.01 to 3.5% by weight, more preferably 0.05 to 2.5% and most preferably 0.1 to 1.5% by weight calculated to the total composition. Most preferred conditioning agents are cationic polymers.

In another preferred form of the invention, aqueous cleansing composition comprises at least one organic solvent such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, polypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzyl alcohol and polypropylene glycols. Total concentration of organic solvents in the shampoo composition should not exceed 5% by weight, preferably in the range of 0.1 to 3%, more preferably 0.5 to 2.5% by weight calculated to total composition.

Aqueous cleansing composition of the present invention may further comprise at least one fatty alcohol of the following formula

R₆-OH

wherein R₆ is straight or branched, saturated or unsaturated alkyl chain with 8 to 24, preferably 10 to 22, more preferably 12 to 18 and most preferably 12 to 16C atoms at a concentration of 0.1 to 5%, preferably 0.1 to 4% and more preferably 0.25 to 3% and most preferably 0.5 to 2.5% by weight calculated to total composition.

Suitable non-limiting preferred examples are decyl alcohol, myristyl alcohol, lauryl alcohol, cetyl alcohol, stearyl alcohol, behenyl alcohol, and arachidyl alcohol and their mixtures. More preferred are decyl alcohol, myristyl alcohol, lauryl alcohol, cetyl alcohol, and stearyl alcohol. Most preferred are decyl alcohol, myristyl alcohol and lauryl alcohol, and their mixtures.

Cleansing composition of the present invention may have pearly appearance. Pearlshiny appearance is achieved with those dispersed in cleansing conditioning compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kinds of mixtures are available commercially.

Solubilizers may be added to the compositions especially when oily substances are chosen as conditioning agents and fragrance oils with highly lipophilic properties. Typical solubilizers may be hydrogenated castor oil known with the trade mark Cremophor CO series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to total composition.

The cleansing composition may contain active ingredients selected from UV filters, moisturisers, sequestering agents, and natural ingredients.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are preferably selected from polycarboxy acids. The preferred one is ethylene diamine tetraacetic acid, EDTA. Typical useful concentration range for sequestering agents is of 0.01 - 2.5% by weight calculated to the total composition.

The UV filters are that oil and water soluble ones for the purpose of protecting hair and hair colour. In other words, anionic and non-ionic, oily, UV filters are suitably used in the compositions of the present invention. Suitable UV-absorbing substances is are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy-benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15. The amount of the UV-absorber ranges typically from about 0.01 % to 2.5%, more preferably from 0.05 % to 1 % by weight, calculated to the total composition.

Natural plant extracts are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, various "Extrapone®" products, and "Herbasol^{R} ". Extracts and the preparation thereof are also described in "Hagers Handbuch der pharmazeutischen Praxis", 4^{th} Ed..

Compositions of the present invention may comprise further at least one compound according to the formula 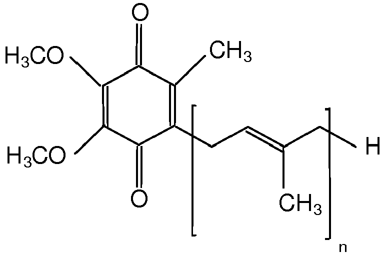 where n is a number from 1 to 10.

The compounds of the above formula are known as Ubiquinone, and also are known as Coenzyme. It should be noted that the compositions of the present invention can certainly comprise more than one ubichinone. Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Cleansing compositions of the present invention can also comprise synthetic mica as a further shine enhancer. Suitable metal oxide or oxides for coating synthetic mica are titanium dioxide, chromium oxide, ferric oxide or mixtures thereof. In the present invention the preferred is synthetic mice coated with titanium dioxide. Such materials are commercially available from Sun Chemical Corporation and known with their INCl names Synthetic Fluorphologopite. Concentration of synthetic mica coated with at least metal oxide or oxides is from 0.001 to 10%, preferably 0.05 to 7.5%, more preferably 0.1 to 5% and most preferably 0.20 to 2.5% by weight calculated to total composition.

Further in a preferred embodiment of the present invention, compositions comprise at least one direct dye and as well deposit dyestuffs onto hair. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye. Certainly the combination of all three dyestuff categories is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87, and mixtures thereof

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium, and mixtures thereof.

Among those, the preferred anionic dyestuffs are Acid Red 52, Acid Violet 2, Acid Red 33, Acid Orange 4, Acid Red 27 and Acid Yellow 10 and their salts, and mixtures thereof. The most preferred anionic dyes are Acid Red 52, Acid Violet 2, Acid Red 33, Acid Orange 4 and Acid Yellow 10, and their salts, and mixtures thereof.

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid, and mixtures thereof.

Concentration of one or more direct dyes in total is in the range of 0.001 to 5% by weight, preferably 0.01 to 4% more preferably 0.05 to 3% and most preferably 0.1 to 2.5% by weight calculated to total composition. The most preferred among the direct dyes is cationic direct dyes.

It is self-understood that the shampoos according to the invention may comprise other substances customarily used in such compositions such as preservatives, fragrances.

The pH of the compositions according to the present invention is in the range of 2.5 to 6.5, more preferably 3 to 6.5 and most preferably 4 to 5.5 measured at ambient temperature with a suitable pH meter.

pH of the compositions is adjusted with acidic and alkaline compounds. Acidic compounds can be inorganic and organic acid or their mixtures. Nonlimiting suitable examples are citric acid, lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Alkaline compounds such as sodium hydroxide can be used to adjust the pH of the compositions.

Aqueous cleansing composition of the present invention preferably comprises one or more thickeners. Suitable ones are ethoxylated polyglyceryl esters with total ethoxy units in the range of 50 to 200 and fatty acyl chain length of 8 to 22 C atoms such as PEG-80 glyceryl cocoate, PEG-90 glyceryl isostearate, PEG-120 glyceryl stearate, PEG-200 glyceryl stearate, PEG-80 glyceryl tallowate, PEG-82 glyceryl tallowate, PEG-130 glyceryl tallowate, and PEG-200 glyceryl tallowate, gylceryl oleate/cocoate and inorganic salt in particular sodium chloride when especially composition comprise alkyl ether sulphate type of surfactants.

Cleansing compositions of the present invention preferably has a viscosity in the range of 500 to 20,000 mPa.s, more preferably 1,000 to 15,000 mPa.s and most preferably 1,500 to 10,000 mPa.s measured at 20°C with a Brookfield viscosimetre using fro example Spindle 5 at appropriate rotation speed.

The following examples are to illustrate the invention, but not to limit. The products according to the invention are prepared by mixing the individual components in water, whereby it is also possible to use pre-mixtures of various ingredients.

### Example 1

**Table I: Comparative aqueous cleansing compositions**

| | % by weight | | | | |
|---|---|---|---|---|---|
| | A | B | C | D | E |
| Sodium cocoyl isethionate | 6 | - | 5 | 6 | 5 |
| Cocamidopropyl betaine | 6 | 9 | 5 | 6 | 5 |
| Coco glucoside | - | - | 3 | - | 3 |
| Sodium lauroyl glutamate | 6 | 9 | 5 | 6 | 5 |
| Polyqauternium-10 | - | - | - | 0.5 | 0.5 |
| Citric acid | q.s to pH 5.0 | | | | |
| Preservative, fragrance | q.s. | | | | |
| Water | q.s. to 100 | | | | |

Shampoo compositions A and C to E are according to the invention and B represents a comparative composition.

Hair tresses were coloured with a commercially available oxidative colouring composition comprising oxidative and direct dyes. The tresses so coloured were washed 10 times with above compositions and before starting the test and afterwards L, a and b values were measured. With the help of the known equation ΔE values were calculated to present colour difference numerically.

Results are presented in Table II.

**Table II: Results of colour wash out test.**

| Composition | **ΔE** |
|---|---|
| A | 4.6 |
| B | 7.2 |
| C | 3.8 |
| D | 4.2 |
| E | 3.6 |

It should be noted that the higher the ΔE value the larger the colour difference between the two tresses. In the opposite, the smaller the ΔE value the smaller the colour difference between the two tresses. From the above results, it is clear that cleansing composition comprising the three surfactants produces lower ΔE values. The following compositions delivered similar colour wash out data.

### Example 2

| | % by weight |
|---|---|
| Sodium lauryl ether sulphate | 5.5 |
| Sodium lauroyl glutamate | 3.5 |
| Decyl glucoside | 2.0 |
| Cocamidopropyl betaine | 3.0 |
| Sodium cocoyl isethionate | 5.0 |
| Polyquaternium-7 | 0.5 |
| PEG-90 glyceryl isostearate | 3.0 |
| Citric acid/sodium hydroxide | q.s. to pH 5.5 |
| Preservative, fragrance | q.s |
| Water | to 100 |

The above shampoo was judged to have rich and creamy foam in a monadic test by the volunteers. It was furthermore mentioned that it foams very quickly. Additionally, the hair washed was excellently combable and had good shine.

### Example 3

| | % by weight |
|---|---|
| Sodium lauryl ether sulphate | 7.0 |
| Sodium lauroyl glutamate | 4.0 |
| Decyl glucoside | 5.0 |
| Cocamidopropyl betaine | 3.0 |
| Sodium cocoyl isethionate | 5.0 |
| Cationic guar | 0.5 |
| PEG-90 glyceryl isostearate | 3.0 |
| Citric acid/sodium hydroxide | q.s. to pH 5.5 |
| Preservative, fragrance | q.s |
| Water | to 100 |

The above composition has excellent creamy rich foam and conditions hair excellently in terms of combability and soft hair feeling.

### Example 4

| | % by weight |
|---|---|
| Sodium lauryl ether sulfate | 3.0 |
| Cocyl glucoside | 3.0 |
| Cocoamidopropyl betaine | 4.0 |
| Sodium lauroyl glutamate | 6.0 |
| Sodium cocoyl isethionate | 3.5 |
| Ethylhexyl glycerine | 1.5 |
| Lauryl alcohol | 0.7 |
| Polyquaternium-10 | 0.5 |
| Dimethicone | 0.5 |
| Ubiquinone | 0.1 |
| Sodium chloride | 1.2 |
| PPG-9 | 2.0 |
| Citric acid/sodium hydroxide | q.s. to pH 5.0 |
| Preservative, fragrance | q.s |
| Water | to 100 |

The above composition improves hair volume, gives hair more elasticity in addition to the excellent creamy foam and conditioning effect in terms of combability, shine and soft hair feeling. Colour wash out effect was observed to be excellent.

### Example 5

| | % by weight |
|---|---|
| Cocoyl polyglucoside | 5.0 |
| Cocamidopropyl betaine | 4.0 |
| Sodium cocoyl glutamate | 7.0 |
| Sodium cocoyl isethionate | 5.5 |
| Decyl glycerine | 1.0 |
| Decyl alcohol | 1.0 |
| Polyquaternium-67 | 0.8 |
| Dimethicone | 0.5 |
| PEG-160 sorbitan triisostearate | 1.0 |
| PPG-9 | 1.2 |
| Basic red 51 | 0.1 |
| Citric acid/sodium hydroxide | q.s. to pH 5.5 |
| Preservative, fragrance | q.s |
| Water | to 100 |

The above composition gives hair a red shine, and additionally delivers excellent conditioning effect in terms of more elasticity, combability, shine and soft hair feeling in addition to the excellent creamy rich foam. The composition foams very quickly.

### Example 6

| | % by weight |
|---|---|
| Sodium lauryl ether sulfate | 5.0 |
| Sodium cocoyl isethionate | 3.5 |
| Laureth - 16 | 3.0 |
| Cocoamidopropyl betaine | 5.0 |
| Sodium cocoyl glutamate | 6.0 |
| Guarhydroxypropyltrimonium chloride | 0.2 |
| Polyqauternium-87 | 0.2 |
| Sodium chloride | 1.0 |
| Heptyl glycerine | 0.7 |
| Myristyl alcohol | 0.5 |
| PPG-9 | 1.0 |
| Trimethyl pentaphenyl trisiloxane | 0.3 |
| Basic yellow 87 | 0.08 |
| Basic red 76 | 0.001 |
| Citric acid/sodium hydroxide | q.s. to pH 5.0 |
| Preservative, fragrance | q.s |
| Water | to 100 |

Excellent conditioning effects were observed in terms of volume, combability, elasticity and managabiliy and additionally an excellent golden blonde shine was observed on light blond hair. Excellent foam quality in terms of speed, volume and creaminess was observed in a monadic test.

### Example 7

| | % by weight |
|---|---|
| Sodium laureth-6 carboxylate | 6.0 |
| Cocyl glucoside | 3.0 |
| Lauramidopropyl betaine | 4.0 |
| Sodium cocoyl glutamate | 5.0 |
| Sodium cocoyl isethionate | 3.5 |
| Polyqauternium-87 | 1.0 |
| PEG-80 glyceryl oleate/cocoate | 1.0 |
| Ethylhexyl glycerine | 1.0 |
| Myristyl alcohol | 1.0 |
| PPG-9 | 1.0 |
| Trimethyl pentaphenyl trisiloxane | 0.3 |
| Basic red 51 | 0.1 |
| Basic orange 31 | 0.05 |
| Citric acid/sodium hydroxide | q.s. to pH 5.0 |
| Preservative, fragrance | q.s |
| Water | to 100 |

Excellent red shine were observed on medium blond hair, in addition to excellent foam characteristics in terms of speed, volume and creaminess in a monadic test.

### Example 8

| | % by weight |
|---|---|
| Sodium lauryl ether sulfate | 6.0 |
| Sodium cocoyl isethionate | 4.5 |
| Laureth - 16 | 2.0 |
| Cocoamidopropyl betaine | 5.0 |
| Sodium cocoyl glutamate | 3.0 |
| Ethylhexyl glycerine | 1.0 |
| Myristyl alcohol | 1.0 |
| Polyquaternium-10 | 0.3 |
| Polyqauternium-87 | 0.2 |
| PEG-90 glyceryl isostearate | 3.5 |
| PPG-9 | 0.7 |
| Carbopol Aqua CC | 5.0 |
| Synthetic fluorphologopite* | 0.5 |
| Citric acid/sodium hydroxide | q.s. to pH 4.7 |
| Preservative, fragrance | q.s |
| Water | to 100 |

| | |
|---|---|
| *: Synthetic fluorphologopite used is commercially available from Sun Chemical Corporation under the trade name SunShine Glitter White with a particle size distribution in the range of 20 to 95 µm. | |

The above composition delivered excellent volume and shine to dark blonde fine hair. Foam characteristics were found to be excellent in terms of volume, speed and creaminess in a monadic test.

### Example 9

| | % by weight |
|---|---|
| Sodium lauryl ether sulfate | 5.0 |
| Cocoyl betaine | 2.0 |
| Cocamidopropyl betaine | 3.0 |
| Decyl glucoside | 1.5 |
| Sodium lauroyl glutamate | 4.0 |
| Sodium cocoyl isethionate | 3.5 |
| Ethylhexyl glycerine | 1.0 |
| Myristyl alcohol | 1.0 |
| Quaternium 80 | 0.5 |
| Polyquaternium-87 | 0.2 |
| Sodium chloride | 1.0 |
| PPG-9 | 1.7 |
| Citric acid/sodium hydroxide | q.s. to pH 5.0 |
| Preservative, fragrance | q.s |
| Water | to 100 |

Above shampoo was found to be excellent volume giving shampoo to fine hair in a monadic test in addition to the excellent foam characteristics as in the previous examples.

With the following examples similar results were found as in the previous examples in hair conditioning and foam characteristics.

### Example 10

| | % by weight |
|---|---|
| Sodium lauryl ether sulphate | 4.0 |
| Cocoyl polyglucoside | 2.0 |
| Cocamidopropyl betaine | 4.0 |
| Sodium cocoyl isethionate | 3.5 |
| Ethylhexyl glycerine | 1.0 |
| Myristyl alcohol | 1.0 |
| Sodium cocoyl glutamate | 3.0 |
| Trimethyl pentaphenyl trisiloxane | 0.3 |
| Polyquaternium-7 | 0.2 |
| Polyquaternium-87 | 0.2 |
| PEG-120 glyceryl stearate | 3.0 |
| PPG-15 | 1.7 |
| Citric acid/sodium hydroxide | q.s. to pH 5.2 |
| Preservative, fragrance | q.s |
| Water | to 100 |

### Example 11

| | % by weight |
|---|---|
| Sodium laureth-6 carboxylate | 8.0 |
| Cocoyl glucoside | 5.0 |
| Cocamidopropyl betaine | 4.0 |
| Sodium cocoyl gluatamate | 3.0 |
| Sodium cocoyl isethionate | 3.5 |
| Ethylhexyl glycerine | 1.0 |
| Myristyl alcohol | 1.0 |
| Polyquaternium-87 | 1.0 |
| PEG-90 glyceryl isostearate | 1.5 |
| Citric acid/sodium hydroxide | q.s. to pH 5.2 |
| Preservative, fragrance | q.s |
| Water | to 100 |

### Example 13

| | % by weight |
|---|---|
| Sodium lauryl ether sulphate | 3.0 |
| Sodium lauryl ether carboxylate | 6.0 |
| Cocoyl polyglucoside | 3.0 |
| Sodium cocoyl glutamate | 3.0 |
| Sodium cocoyl isethionate | 3.5 |
| Cocamidopropyl betaine | 3.0 |
| Polyquaternium-87 | 0.8 |
| Dimethicone | 0.5 |
| PEG-90 glyceryl isostearate | 3.0 |
| Basic red 51 | 0.1 |
| Citric acid/sodium hydroxide | q.s. to pH 5.5 |
| Preservative, fragrance | q.s |
| Water | to 100 |

### Example 14

| | % by weight |
|---|---|
| Sodium lauryl ether sulfate | 9.0 |
| Laureth - 16 | 3.0 |
| Cocamidopropyl betaine | 3.0 |
| Sodium lauroyl glutamate | 3.0 |
| Sodium cocoyl isethionate | 3.5 |
| Ethylhexyl glycerine | 1.0 |
| Polyquaternium-87 | 1.0 |
| Sodium chloride | 1.3 |
| PPG-20 | 0.8 |
| Trimethyl pentaphenyl trisiloxane | 0.2 |
| Basic yellow 87 | 0.10 |
| Basic red 76 | 0.01 |
| Citric acid/sodium hydroxide | q.s. to pH 6.0 |
| Preservative, fragrance | q.s |
| Water | to 100 |

## Claims

1. Aqueous cleansing composition for keratin fibres especially for human hair **characterised in that** it comprises at least one mono ester anionic surfactant selected from the compounds according to the general structure
R₃₀ C(O) O R₃₁ SO₃ M
wherein R₃₀ is a saturated or unsaturated, straight or branched alkyl chain with 9 to 17 C atoms, R₃₁ is a methyl or straight or branched alkyl chain with 1 to 4 C atoms and M is ammonium or an alkali metal, preferably sodium, at least one anionic amide surfactant selected from the compounds according to the general structure 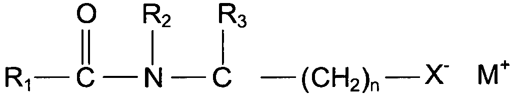 wherein R₁ is a saturated or unsaturated, straight or branched alkyl chain with 7 to 17 C atoms, R₂ is H, R₃ is H, COO- M⁺, CH₂COO⁻ M⁺ or COOH, n is 0 to 2, X is COO⁻ or SO₃⁻ and M is independent from each other H, sodium or potassium, and at least one amphoteric amide surfactant, wherein the composition has a pH in the range of 2.5 to 6.5

2. The composition according to claim 1 **characterized in that** it has a pH in the range of 3 to 5.5.

3. The composition according to claims 1 and 2 **characterised in that** at least one anionic amide surfactant is selected from taurate, glutamate and aspartate surfactants.

4. The composition according to any of the preceding claims **characterised in that** at least one amphoteric amide surfactant is selected from the compounds according to the general structure 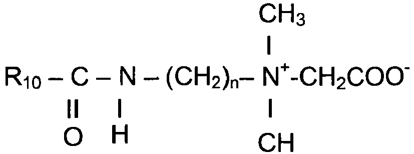 wherein R₁₀ is a straight or branched, saturated or unsaturated C₈-C₂₂-alkyl group, preferably C₁₀-C₁₈-alkyl group and more preferably C₁₀-C₁₄-alkyl group and n is 1 to 3.

5. The composition according to any of the preceding claims **characterised in that** it comprises surfactant at a total concentration of 5 to 50%, preferably 7.5 to 40%, more preferably 8 to 30% and most preferably 10 to 25% and particularly 10 to 20% by weight, calculated to the total of the composition.

6. The composition according to any of the preceding claims **characterised in that** it comprises at least one mono ester anionic surfactant at a concentration of 1 to 15% by weight, at least one anionic amide surfactant at a concentration of 1 to 15% by weight and at least one amphoteric amide surfactant at a concentration of 1 to 15% by weight, wherein all values are calculated to the total content of the composition.

7. The composition according to any of the preceding claims **characterised in that** at least one mono ester anionic surfactant is sodium or ammonium cocoyl isethionate.

8. The composition according to any of the preceding claims **characterised in that** it comprises at least one non-ionic surfactant.

9. The composition according to any of the preceding claims **characterised in that** it comprises at least one hair conditioning agent.

10. The composition according to claim 9 **characterised in that** the hair conditioning agent is selected from cationic polymers.

11. The composition according to any of the preceding claims **characterised in that** it comprises at least one direct dye.

12. Use of composition according to any of the preceding claims for cleansing and conditioning hair.

13. Use of composition according to preceding claims 1 to 10 for reducing colour fading and/or colour wash out from artificially coloured hair.

14. Kit for treating hair **characterised in that** it comprises two or more compositions wherein at least one of the compositions is a composition according to claims 1 to 10.

## Patentansprüche

1. Wässrige Reinigungszusammensetzung für Keratinfasern, insbesondere für menschliches Haar, **dadurch gekennzeichnet, dass** sie mindestens ein anionisches Monoester-Tensid, ausgewählt aus den Verbindungen der allgemeinen Struktur
R₃₀ C(O) O R₃₁ SO₃ M
wobei R₃₀ für eine gesättigte oder ungesättigte, lineare oder verzweigte Alkylkette mit 9 bis 17 C-Atomen steht, R₃₁ für ein Methyl oder eine lineare oder verzweigte Alkylkette mit 1 bis 4 C-Atomen steht und M für Ammonium oder ein Alkalimetall, vorzugsweise Natrium, steht, mindestens ein anionisches Amid-Tensid, ausgewählt aus den Verbindungen der allgemeinen Struktur 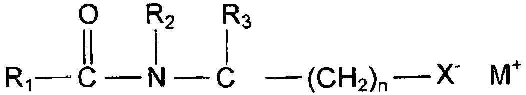 wobei R₁ für eine gesättigte oder ungesättigte, lineare oder verzweigte Alkylkette mit 7 bis 17 C-Atomen steht, R₂ für H steht, R₃ für H, COO⁻ M⁺, CH₂COO⁻ M⁺ oder COOH steht, n 0 bis 2 ist, X für COO⁻ oder SO₃⁻ steht und M jeweils unabhängig für H, Natrium oder Kalium steht, und mindestens ein amphoteres Amid-Tensid aufweist, wobei die Zusammensetzung einen pH-Wert im Bereich von 2,5 bis 6,5 aufweist.

2. Zusammensetzung nach Anspruch 1, **dadurch gekennzeichnet, dass** sie einen pH-Wert im Bereich von 3 bis 5,5 aufweist.

3. Zusammensetzung nach Anspruch 1 und 2, **dadurch gekennzeichnet, dass** mindestens ein anionisches Amid-Tensid aus Taurat-, Glutamat- und Aspartat-Tensiden ausgewählt ist.

4. Zusammensetzung nach einem der vorhergehenden Ansprüche, **dadurch gekennzeichnet, dass** mindestens ein amphoteres Amid-Tensid ausgewählt ist aus Verbindungen der allgemeinen Struktur 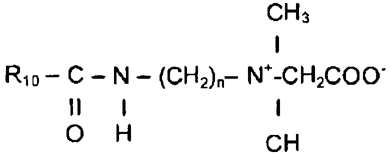 wobei R₁₀ für eine lineare oder verzweigte, gesättigte oder ungesättigte C₈-C₂₂-Alkylgruppe, vorzugsweise für eine C₁₀-C₁₈-Alkylgruppe und insbesondere für eine C₁₀-C₁₄-Alkylgruppe steht und n 1 bis 3 ist.

5. Zusammensetzung nach einem der vorhergehenden Ansprüche, **dadurch gekennzeichnet, dass** sie Tensid in einer Gesamtkonzentration von 5 Gew.-% bis 50 Gew.-%, vorzugsweise 7,5 Gew.-% bis 40 Gew.-%, mehr bevorzugt 8 Gew.-% bis 30 Gew.-% und am meisten bevorzugt 10 Gew.-% bis 25 Gew.-% und insbesondere 10 Gew.-% bis 20 Gew.-% aufweist, bezogen auf die Gesamtzusammensetzung.

6. Zusammensetzung nach einem der vorhergehenden Ansprüche, **dadurch gekennzeichnet, dass** sie mindestens ein anionisches Monoester-Tensid in einer Konzentration von 1 Gew.-% bis 15 Gew.-%, mindestens ein anionisches Amid-Tensid in einer Konzentration von 1 Gew.-% bis 15 Gew.-% und mindestens ein amphoteres Amid-Tensid in einer Konzentration von 1 Gew.-% bis 15 Gew.-% aufweist, wobei alle Werte auf den Gesamtinhalt der Zusammensetzung bezogen sind.

7. Zusammensetzung nach einem der vorhergehenden Ansprüche, **dadurch gekennzeichnet, dass** mindestens ein anionisches Monoester-Tensid Natrium- oder Ammoniumcocoylisethionat ist.

8. Zusammensetzung nach einem der vorhergehenden Ansprüche, **dadurch gekennzeichnet, dass** sie mindestens ein nichtionisches Tensid aufweist.

9. Zusammensetzung nach einem der vorhergehenden Ansprüche, **dadurch gekennzeichnet, dass** sie mindestens ein Haarkonditionierungsmittel aufweist.

10. Zusammensetzung nach Anspruch 9, **dadurch gekennzeichnet, dass** das Haarkonditionierungsmittel aus kationischen Polymeren ausgewählt ist.

11. Zusammensetzung nach einem der vorhergehenden Ansprüche, **dadurch gekennzeichnet, dass** sie mindestens einen Direktfarbstoff aufweist.

12. Verwendung einer Zusammensetzung nach einem der vorhergehenden Ansprüche zum Reinigen und Konditionieren von Haar.

13. Verwendung einer Zusammensetzung nach einem der vorhergehenden Ansprüche 1 bis 10 zum Verringern des Farbschwunds und/oder der Farbauswaschung bei künstlich gefärbtem Haar.

14. Kit zum Behandeln von Haar, **dadurch gekennzeichnet, dass** es zwei oder mehr Zusammensetzungen aufweist, wobei mindestens eine der Zusammensetzungen eine Zusammensetzung nach Anspruch 1 bis 10 ist.

## Revendications

1. Composition aqueuse de nettoyage pour des fibres de kératine, notamment pour des cheveux humains, **caractérisée en ce qu'**elle comprend au moins un tensioactif anionique de type mono ester choisi parmi les composés selon la structure générale
R₃₀ C(O) O R₃₁ SO₃ M
où R₃₀ est une chaîne alkyle saturée ou insaturée, linéaire ou ramifiée, avec 9 à 17 atomes C, R₃₁ est un groupe méthyle ou une chaîne alkyle linéaire ou ramifiée avec 1 à 4 atomes C et M est un ion ammonium ou de métal alcalin, de préférence de sodium, au moins un tensioactif anionique de type amide choisi parmi les composés selon la structure générale 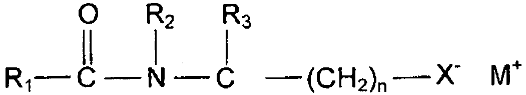 où R₁ est une chaîne alkyle saturée ou insaturée, linéaire ou ramifiée, avec 7 à 17 atomes C, R₂ est un atome H, R₃ est un atome H, un groupe COO⁻M⁺, un groupe CH₂COO⁻ M⁺ ou COOH, n est un nombre de 0 à 2, X est un ion COO⁻ ou SO₃⁻ et M est indépendant de tout autre atome H, sodium ou potassium, et au moins un tensioactif amphotère de type amide, où la composition a un pH dans la gamme de 2,5 à 6,5.

2. Composition selon la revendication 1, **caractérisée en ce qu'**elle a un pH dans la gamme de 3 à 5,5.

3. Composition selon les revendications 1 et 2, **caractérisée en ce qu'**au moins un tensioactif anionique est choisi parmi des tensioactifs de type amide parmi un taurate, un glutamate et un aspartate.

4. Composition selon l'une quelconque des revendications précédentes, **caractérisée en ce qu'**au moins un tensioactif amphotère de type d'amide est choisi parmi les composés selon la structure générale 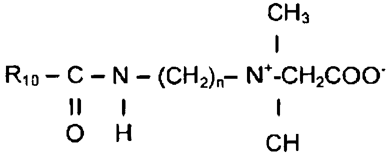 où R₁₀ est un groupe alkyle en C₈-C₂₂ linéaire ou ramifié, saturé ou insaturé, de préférence un groupe alkyle en C₁₀-C₁₈ et plus préférentiellement un groupe alkyle en C₁₀-C₁₄ et n est un nombre de 1 à 3.

5. Composition selon l'une quelconque des revendications précédentes, **caractérisée en ce qu'**elle comprend du tensioactif à une concentration totale de 5 à 50 %, de préférence de 7,5 à 40 %, plus préférentiellement de 8 à 30 % et idéalement de 10 à 25 % et particulièrement préféré de 10 à 20 % en poids, calculée par rapport à la composition totale.

6. Composition selon l'une quelconque des revendications précédentes, **caractérisée en ce qu'**elle comprend au moins un tensioactif anionique de type mono ester à une concentration de 1 à 15 % en poids, au moins un tensioactif anionique de type amide à une concentration de 1 à 15 % en poids et au moins un tensioactif amphotère de type amide à une concentration de 1 à 15 % en poids, où toutes les valeurs sont calculées par rapport à la teneur totale de la composition.

7. Composition selon l'une quelconque des revendications précédentes, **caractérisée en ce qu'**au moins un tensioactif anionique de type mono ester est du cocoyl iséthionate de sodium ou d'ammonium.

8. Composition selon l'une quelconque des revendications précédentes, **caractérisée en ce qu'**elle comprend au moins un tensioactif non ionique.

9. Composition selon l'une quelconque des revendications précédentes, **caractérisée en ce qu'**elle comprend au moins un agent de conditionnement des cheveux.

10. Composition selon la revendication 9, **caractérisée en ce que** l'agent de conditionnement des cheveux est choisi parmi des polymères cationiques.

11. Composition selon l'une quelconque des revendications précédentes, **caractérisée en ce qu'**elle comprend au moins un colorant direct.

12. Utilisation de la composition selon l'une quelconque des revendications précédentes pour le nettoyage et le conditionnement des cheveux.

13. Utilisation de la composition selon les revendications précédentes 1 à 10 pour une réduction du caractère fade de la couleur et/ou d'un délavage de la couleur, pour des cheveux colorés artificiellement.

14. Kit pour le traitement des cheveux **caractérisé en ce qu'**il comprend deux ou plusieurs compositions, où au moins l'une des compositions est une composition selon les revendications 1 à 10.
